# EUROPEAN PATENT APPLICATION

(11) **EP 1 916 257 A1**
(43) Date of publication of application: **30.04.2008**
(21) Application number: 06022548.9
(22) Date of filing: 27.10.2006
(51) Int. Cl.: C07K 7/06, A61K 39/00, A61P 35/00

(54) **GD2 peptide mimotopes for anticancer vaccination**

(71) Applicant: Charité-Universitätsmedizin Berlin, 10117 Berlin (DE)
(72) Inventor: Lode, Holger, Dr., 13469 Berlin (DE); Fest, Stefan, Dr., 10245 Berlin (DE); Bleeke, Matthias, 10551 Berlin (DE); Gaedicke, Gerhard, Prof., 10829 Berlin (DE); Volkmer, Rudolf, Dr., 12621 Berlin (DE)
(74) Representative: Zwicker, Jörk

(57) **Abstract**

The present invention relates to a disialoganglioside GD2 peptide mimotope capable of eliciting an immune response against GD2, polynucleotides encoding disialoganglioside GD2 peptide mimotopes, vectors comprising such polynucleotides, cells expressing them and uses of polypeptides, polynucleotides, vectors and cells for the production of a medicament for the treatment of tumors of neuroectodermal origin.
The GD2 mimotopes of the invention have one of the following amino acid sequences:
GRLX₁X₂VPDLE, wherein X₁ is selected from K, H, S, N and X₂ from M, I, L;
DGX₃WLSX₄GSW, wherein X₃ is selected from G, W, E, D and X₄ from E, K, N.
They are preferably flanked by two disulfide-bridged cysteines and formulated as DNA vaccines.

## Description

The present invention relates to a disialoganglioside GD2 peptide mimotope capable of eliciting an immune response against GD2, polynucleotides encoding disialoganglioside GD2 peptide mimotopes, vectors comprising such polynucleotides, cells expressing them and uses of polypeptides, polynucleotides, vectors and cells for the production of a medicament for the treatment of tumors of neuroectodermal origin.

### BACKROUND OF THE INVENTION

Gangliosides are sialylated glycosphingolipids which have been implicated as tumor-associated membrane antigens in tumors of neuroectodermal origin (Hakamori and Kannagi (1983) Int. J. Cancer 71:231-251). Examples of such gangliosides are GM3, GD3, GM2 and GD2.

The disialoganglioside antigen GD2 consists of a backbone of oligosaccharides flanked by sialic acid and lipid residues (Cheresh, D. A. (1987) Surv. Synth. Pathol. Res. 4:97). The antigen is highly expressed on tumor cells of neuroectodermal origin including melanoma, neuroblastoma, and small cell carcinoma of the lung (Hakomori, S.-I. and R. Kannagi (1983) J. Natl. Cancer Inst. 71:231; Cheresh, D. A, et al. (1986) Cancer Res. 46:5112; Mujoo, K, et al. (1987) Cancer Res. 47:1098).

GD2 has been shown to be highly expressed on most melanoma cells and its expression in normal cells is mostly restricted to the brain. Accordingly, GD2 has been a candidate for vaccine development, especially in combination with other gangliosides (Hamilton et al. (1993) Int. J. Cancer 53:566-573). The GD2 antigen has also been the target of a number of therapeutic monoclonal antibody trials that have shown some *in vivo* antitumor effects (Saleh, M. N, et al. (1992) Human Antibodies and Hybridomas 3(1):19; Saleh, M. N. et al. (1992) Cancer Res. 52:4342; Cheung, N.-K. V, et al. (1992) J. Clin. Oncol. 10:671). GD2 has been used for passive immunotherapy with two different monoclonal anti-GD-2 antibodies (Mab), namely the murine Mab 3F8 and the human mouse chimeric Mab ch14.18 (Cheung N.K. et al. (1998) J Clin. Oncol. 16:3053; Handgretinger R. et al. (1995) Eur J. Cancer 31A:261; Yu A.L. et al. (1998) J. Clin. Oncol. 16:2169 and Simon T, et al. (2004) J Clin. Oncol. 22:3549), which is entering a European phase III clinical trail (HR-NBL-1/ESIOP) (Zeng Y. et al. (2005) Mol. Immunol. 42:1311).

Although gangliosides would be useful as vaccines, they are difficult to produce. Furthermore, some gangliosides are only weakly immunogenic. Those that are immunogenic may not be found on all tumor cells. In humans, the GD2 antigen is weakly immunogenic and generally induces T cell-independent humoral immune responses (Tai, T. et al, 1985, Int. J. Cancer 35:607; Portoukalian et al., Int. J. Cancer, 49:893-899 (1991)).

In order to improve efficacy of passive immunotherapy directed against GD2, active vaccination was investigated using GD2-keyhole limpet hemocyanin (KLH) conjugates, anti-idiotypic antibodies and its encoding plasmid (Chapman P.B. et al. (2000) Clin. Cancer Res. 6:4658; Foon K.A. et al. (2000) Clin. Oncol. 18:376; Sen G. et al. (1998) J. Immunother. 21:75 and Zeytin, H.E. (2000) Cancer Gene Ther. 7:1426). Anti-idiotypic antibodies have been studied as potential vaccines against pathogenic organisms (Kennedy, R. C. et al. (1986) Science 232:220; Reagan, K. J. et al., (1983) J Virol. 48:660; McNamara, M. K. et al. (1984) Science 226:1325; Sachs, D. L. et al. (1982) J Exp. Med. 155:1108) and malignant tumors (Chen, Z. J. et al. (1991) J Immunol. 147(3):1082; Dunn, P. L. et al. (1987) J Immunol. 60:181-187; Herlyn, D. et al. (1987) Proc. Natl. Acad. Sci. USA 84:8055; Chattopadhyay, P. et al. (1991) Cancer Res. 51:3183). In animal studies, murine anti-idiotypic antibodies have demonstrated antigen-specific responses across species (xenogeneic model) (Chapman, P. B. and A. N. Houghton (1991) J. Clin. Invest. 88:186) and within the same inbred species (syngeneic model) (Gaulton, G. N. et al. (1986) J Immunol. 137:2930; Chen, Z. J. et al. (1991) J Immunol. 147(3):1082; Dunn, P. L. et al. (1987) J Immunol. 60:181-187; Yamamoto, S. et al., J Natl. Cancer Inst. 82:1757). The ability of "internal image" anti-idiotypic antibodies to function as immunogens in a syngeneic system implies that the unique CDR epitopes of the anti-idiotypic antibody can be seen as foreign even by animals that otherwise share a very similar antibody repertoire.

These studies revealed a moderate improvement of disease outcome, none of these active immunization approaches have entered extensive clinical application so far. One general obstacle to overcome in the generation of a vaccine inducing an anti-GD2 immune response is the antigen itself, since GD2 is a glycolipid, which evokes poor immune responses and functions as a T-cell independent antigen (Mond J. J. et al. (1995) Curr. Opin. Immunol. 7:349-54). One possibility to involve T-cells and provide for effective B-cell help followed by the generation of pro-inflammatory CD3⁺/CD4⁺ T helper (TH₁) cells, is to change the nature of the antigen from glycolipid to peptide with a peptide-mimotope approach (Grothaus M.C. et al. (2000) Vaccine 18:1253) In previous work we described for the first time the identification of 13 independent circular GD2 decapeptide mimotopes (Forster-Waldl E, et al. (2005) Mol. Immunol. 42:319). This was accomplished by biopanning a phage-display library, presenting 1.04 x 10⁹ different circular peptide sequences (Mazzucchelli L et al. (1999) Blood 93:1738) with the anti-GD2 Mab ch14.18. Similar work has been reported (Bolesta E, et al. (2005) Cancer Res. 65: 3410) on the identification of GD2 peptide mimotopes using phage-display library presenting linear peptides. The use of DNA vaccines encoding for these linear peptides was without the identification of in vivo efficacy in an immunocompetent animal model. Thus, there is still a need for easy to produce and administer compounds that expose epitopes structurally related to GD2, which are stable and easier to administer than, e.g. antibody vaccines. The present inventors have solved this problem by providing decapeptide mimotopes with particular high affinity to GD2 specific antibody ch14.18 and, which are, thus, particularly suitable to be used in active vaccination approaches.

### SUMMARY OF THE INVENTION

In a first aspect the present invention relates to a polypeptide comprising a GD2 peptide mimotope having the amino acid sequence
GRLX₁X₂VPDLE,
wherein
X₁ is K, H, S, or N
X₂ is M, I or L
or
DGX₃WLSX₄GSW
wherein
X₃ is G, W, E, or D
X₄ is E, K, or N
or a variant thereof, wherein the variant comprises one or two further amino acid changes and exhibits a dissociation constant (K_{D}) to an antibody comprising the mature Ig light chain sequence derived from the light chain pre-protein according to SEQ ID 1 and the mature Ig heavy chain sequence derived from the heavy chain pre-protein according to SEQ ID NO. 2 of 150 x 10⁻⁶ M or less.

In a preferred embodiment the present invention relates to a polypeptide comprising a GD2 peptide mimotope, wherein the GD2 peptide mimotope is producible by a method comprising the steps of synthesizing a polypeptide comprising a GD2 peptide mimotope having an amino acid sequence wherein one or two amino acid(s) from the amino acid sequence selected from the group consisting of SEQ ID NO. 3 to 14 has(have) been changed and selecting a polypeptide exhibiting a K_{D} to an antibody having an Ig light chain sequence according to SEQ ID 1 and an Ig heavy chain sequence according to SEQ ID NO. 2 of 150 x 10⁻⁶ M or less.

In a further aspect the present invention relates to a polynucleotide comprising a nucleotide sequence encoding a polypeptide of the present invention.

In a further aspect the present invention relates to a vector containing the polynucleotide of the present invention.

In a further aspect the present invention relates to a host cell genetically engineered with the polynucleotide of the present invention or the vector of the present invention.

In a further aspect the present invention relates to a pharmaceutical composition comprising a polypeptide of the present invention, a polynucleotide of the present invention, a vector of the present invention or a host cell of the present invention and a pharmaceutically acceptable adjuvant.

In a further aspect the present invention relates to the use of a polypeptide of the present invention, a polynucleotide of the present invention, a vector of the present invention or a host cell of the present invention for the production of a medicament for the treatment of tumors of neuroectodermal origin.

### DETAILED DESCRIPTION

### Definitions

Before the present invention is described in detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art.
Preferably, the terms used herein are defined as described in "A multilingual glossary of biotechnological terms: (IUPAC Recommendations)", Leuenberger, H.G.W, Nagel, B. and Klbl, H. eds. (1995), Helvetica Chimica Acta, CH-4010 Basel, Switzerland).

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step.

Several documents are cited throughout the text of this specification. Each of the documents cited herein (including all patents, patent applications, scientific publications, manufacturer's specifications, instructions, etc.), whether supra or infra, are hereby incorporated by reference in their entirety. Nothing herein is to be construed as an admission that the invention is not entitled to antedate such disclosure by virtue of prior invention.

Polypeptide comprising a GD2 peptide mimotope having the amino acid sequence
GRLX₁X₂VPDLE,
wherein
X₁ is K, H, S, or N
X₂ is M, I or L
or
DGX₃WLSX₄GSW
wherein
X₃ is G, W, E, or D
X₄ is E, K, or N
or a variant thereof, wherein the variant comprises one or two further amino acid substitutions and exhibits a K_{D} to an antibody comprising the mature Ig light chain sequence derived from the light chain pre-protein according to SEQ ID 1 and the mature Ig heavy chain sequence derived from the heavy chain pre-protein according to SEQ ID NO. 2 of 150 x 10⁻⁶ M or less. The single letter code used for designating amino acids has its usual meaning. The term "variant" of a GD2 peptide mimotope as used in the present application is to be understood as a polypeptide which differs in comparison to the polypeptide from which it is derived by one or two changes in the amino acid sequence. The changes in the amino acid sequence may be amino acid exchanges, insertions, deletions, N-terminal truncations, or C-terminal truncations, or any combination of these changes, which may occur at one or two sites. Preferably, the change is an amino acid exchange occurring at one or two sites. The amino acid exchanges may be conservative or non-conservative. In preferred embodiments the variant of a GD2 peptide mimotope comprises one or two conservative amino acid exchanges. "Non-conservative substitutions" or "non-conservative amino acid exchanges" are defined as exchanges of an amino acid by another amino acid listed in a different group of the six standard amino acid groups shown below:
(1) hydrophobic: M, A, V, L, or I;
(2) neutral hydrophilic: C, S, or T;
(3) acidic: D, or E;
(4) basic: N, Q, H, K, or R;
(5) residues that influence chain orientation: G, or P and
(6) aromatic: W, Y, or F.

Conservative exchanges are defined as exchanges of an amino acid by another amino acid listed within the same group of the six standard amino acid groups shown above.

The K_{D} of a polypeptide comprising a GD2 peptide mimotope towards an antibody comprising an Ig light chain sequence according to SEQ ID 1 and an Ig heavy chain sequence according to SEQ ID NO. 2 can be determined by any art known method to determine binding affinities, preferably the K_{D} is determined using the Biacore system. The dissociation constant (K_{D}) is preferably calculated with the curve fitting program supplied by Biacore software. Preferably, the K_{D} of the polypeptide comprising a GD2 peptide mimotope 150 x 10⁻⁶ M, preferably of 140 x 10⁻⁶ M or less, 130 x 10⁻⁶ M or less, 120 x 10⁻⁶ M or less, 110 x 10⁻⁶ M or less, 100 x 10⁻⁶ M or less, 90 x 10⁻⁶ M or less, 80 x 10⁻⁶ M or less, 70 x 10⁻⁶ M or less, 60 x 10⁻⁶ M or less, 50 x 10⁻⁶ M or less, 40 x 10⁻⁶ M or less, 30 x 10⁻⁶ M or less, and most preferably 20 x 10⁻⁶ M or less. The K_{D} is preferably determined between 15 and 40°C, more preferably between 20 and 30°C and most preferably at about 25°C.

The antibody used for the assessment of K_{D} is the human mouse chimeric anti-GD2 antibody ch14.18, which comprises a heavy and light chain derived from the pre-protein sequences set out in SEQ ID NO. 1 and 2, respectively. It is known in the art that functional antibodies comprise a light and a heavy chain, which originate from two pre-proteins, which are proteolytically cleaved during antibody maturation. Someone of skill in the art will be capable of producing these antibodies without undue burden on the basis of the nucleotide sequence provided herein. It is understood that it would also be possible to test the K_{D} of various fragments of this antibody, which will have binding activities similar to ch14.18 in as long as the variable chains of the light and heavy chain, respectively, have not been significantly altered.

In a preferred embodiment the GD2 peptide mimotope comprised in the polypeptide of the present invention has an amino acid sequence selected from the group consisting of GRLKMVPDLE (SEQ ID NO. 3), GRLHMVPDLE (SEQ ID NO. 4), GRLSMVPDLE (SEQ ID NO. 5), GRLKLVPDLE (SEQ ID NO. 6), GRLHLVPDLE (SEQ ID NO. 7), GRLSLVPDLE (SEQ ID NO. 8), GRLNLVPDLE (SEQ ID NO. 9), DGGWLSKGSW (SEQ ID NO. 10), DGGWLSEGSW (SEQ ID NO. 11), DGWWLSEGSW (SEQ ID NO. 12), DGDWLSKGSW (SEQ ID NO. 13), and DGGWLSNGSW (SEQ ID NO. 14) or is a variant thereof, wherein the variant comprises one or two further amino acid changes, preferably exchange(s), and exhibits a K_{D} to an antibody comprising the mature Ig light chain sequence derived from the light chain pre-protein according to SEQ ID 1 and the mature Ig heavy chain sequence derived from the heavy chain pre-protein according to SEQ ID NO. 2 of 150 x 10⁻⁶ M or less, preferably of 140 x 10⁻⁶ M or less, preferably of 130 x 10⁻⁶ M or less, preferably of 120 x 10⁻⁶ M or less, preferably of 110 x 10⁻⁶ M or less, preferably of 100 x 10⁻⁶ M or less, preferably of 90 x 10⁻⁶ M or less, preferably of 80 x 10⁻⁶ M or less, preferably of 70 x 10⁻⁶ M or less, preferably of 60 x 10⁻⁶ M or less, preferably of 50 x 10⁻⁶ M or less, preferably of 40 x 10⁻⁶ M or less, preferably of 30 x 10⁻⁶ M or less, and most preferably 20 x 10⁻⁶ M or less. A particularly preferred mimotope is GRLHLVPDLE (SEQ ID NO. 7).

It has been noted that an increased binding affinity is generally observed, if the short GD2 peptide mimotope comprised within the peptide, preferably a 8, 9, 10, 11 or 12 mer, preferably a 10 mer is cyclic. Accordingly, preferred polypeptides of the present invention comprise at least one cyclization moiety N- and C-terminally of the GD2 peptide mimotope. The cyclization moieties are capable of reacting with each other to form a covalent or non-covalent, preferably a covalent bond. Accordingly, suitable cyclization moieties comprise reactable residues like sulfhydryl groups, epoxy groups, etc. which are readily known to someone of skill in the art. It is preferred that the cyclization moieties are amino acids incorporated N- and C-terminally of the GD2 mimotope in the polypeptide chain, like e.g. Cys or Homo Cys residues, or are residues which are attached after recombinant or synthetic production of the polypeptide comprising or consisting of the GD2 peptide mimotope, by chemical reactions. Accordingly, preferred cyclization moieties comprise Cys or Homo Cys or the cyclisation moity is selected from a combination of the following pairs (one at the N-terminus the other at the C-terminus or vice versa): jodo-, chloro- or bromoacetic acid and Cys, jodo-, chloro- or bromacetic acid and Homo-Cys, 2-maleimidoacetic acid and Cys, 2-maleimidoacetic acid and Homo-Cys, 3-maleimidopropionic acid and Cys, 3-maleimidopropionic acid and Homo-Cys, 6-maleimidocaproic acid and Cys, 6-maleimidocaproic acid and Homo-Cys or combinations of the pairs: C-terminal Glu and any N-terminal canonical amino acid, C-terminal Asp and any N-terminal canonical amino acid.However, since it is preferred that the polypeptides of the present invention comprising a GD2 mimotope are expressed from a polynucleotide encoding them, it is preferred that the cyclization moiety is Cys or Homo Cys, which can be readily incorporated in a polypeptide chain. Preferably, at least one more preferably both of the N- and C-terminally located cyclization moieties are directly linked to the GD2 peptide mimotope. In a particular preferred embodiment one or more cysteine or Homo Cys residues are located immediately at the N- and at the C-terminus of the GD2 peptide mimotope. Thus, in a particular preferred embodiment the GD2 peptide mimotopes, preferably according to SEQ ID NO. 3 to 14 are N-and C-terminally flanked by Cys residues. Examples of such preferred Cys-flanked mimotopes are CGRLKMVPDLEC (SEQ ID NO. 15), CDGGWLSKGSWC (SEQ ID NO.16) and CGRLHLVPDLEC (SEQ ID NO. 17).

The GD2 peptide mimotopes of the invention are capable of eliciting an immune response specific to GD2. To foster that immune response it is desirable that further immunogenic compounds are covalently or non-covalently attached to the polypeptide of the invention. Examples of such immunogens, which can be part of a covalent of non-covalent conjugate are well known in the art and comprise without limitation unmethylated DNA comprising CpG dinucleotides (CpG motif), in particular CpG ODN with phosphorothioate (PTO) backbone (CpG PTO ODN) or phosphodiester (PO) backbone (CpG PO ODN); gel-like precipitates of aluminum hydroxide (alum); bacterial products from the outer membrane of Gram-negative bacteria, in particular monophosphoryl lipid A (MPLA), lipopolysaccharides (LPS), muramyl dipeptides and derivatives thereof; synthetic lipopeptide derivatives, in particular Pam₃Cys; lipoarabinomannan; peptidoglycan; zymosan; heat shock proteins (HSP), in particular HSP 70; dsRNA and synthetic derivatives thereof, in particular Poly I:poly C; polycationic peptides, in particular poly-L-arginine; taxol; fibronectin; flagellin; keyhole limpet hemocyanine (KLH), imidazoquinoline; cytokines with adjuvant activity, in particular GM-CSF, interleukin- (IL-)2, IL-6, IL-7, IL-18, type I and II, interferons, in particular interferon-gamma, TNF-alpha; saponins and derivatives thereof, in particular the immunostimulatory fragments from saponins; polyphosphazene; N-(2-Deoxy-2-L-leucylamino-β-D-glucopyranosyl)-N-octade-cyldodecanoylamide hydroacetate (BAY R1005), 25-dihydroxyvitamin D3 (calcitriol); DHEA; murametide [MDP(Gln)-OMe]; murapalmitine; polymers of lactic and/or glycolic acid; polymethyl methacrylate; sorbitan trioleate; squalane; stearyl tyrosine; squalene; theramide, synthetic oligopeptides, in particular MHCII-presented peptides. It is particularly preferred that the GD2 peptide mimotope and the immunogen is coexpressable from one encoding polynucleotide and, accordingly, in a preferred embodiment the polypeptide of the present invention further comprises an immunogen, preferably selected from the group consisting of T1 epitope of HIV gp 120; zymosan; heat shock proteins (HSP), in particular HSP 70; polycationic peptides, in particular poly-L-arginine; fibronectin; flagellin; cytokines with adjuvant activity, in particular GM-CSF, interleukin- (IL-)2, IL-6, IL-7, IL-11, IL-18, type I and II, interferons, in particular interferon-gamma, TNF-alpha; synthetic oligopeptides presented by MHCII. A large number of such oligopeptides has been described in the prior art.

As it is preferred in one embodiment that the immunogen is coexpressed with the GD2 peptide mimotope the GD2 polypeptide is preferably linked to the immunogen via a peptide linker, i.e. the immunogen and the GD2 peptide mimotope is a fusion protein.

To elicit an appropriate T cell response it is often required that a polypeptide to which an immune reaction is to be elicited is presented by the MHC system. To that end it may be advantageous that the polypeptides of the invention are capable to enter antigen presenting cells as, e.g. dendritic cells (DCs) or macrophages. Accordingly, the polypeptide of the present invention comprises in one preferred embodiment a cellular uptake enhancing moiety. Such a moiety is generally recognized and bound by an extracellular receptor and subsequently internalized. A large number of such moieties have been described in the prior art. Preferred examples of such cellular uptake enhancing moieties comprise without limitation human immune deficiency virus (HIV) Tat and cellular uptake enhancing fragments and variants thereof, herpes simplex virus (HSV) VP22 tegument protein and cellular uptake enhancing fragments and variants thereof, homeoproteins including Drosophila homeotic protein antennapedia (ANTp), Engrailed 1 (En1), Engrailed 2 (En2), Hoxa-5, Hoxc-8, Hoxb-4 and KNOTTED-1 and cellular uptake enhancing fragments and variants thereof. Whether a fragment or variant is still uptake enhancing or not can easily be determined with art known methods involving, e.g. the use of radioactively or fluorescently labelled variants or fragments and the subsequent incubation of these variants or fragments with DCs or macrophages.

In a further preferred embodiment the present invention relates to a polypeptide comprising a GD2 peptide mimotope, wherein the GD2 peptide mimotope is producible by a method comprising the steps of synthesizing a polypeptide comprising a GD2 peptide mimotope having an amino acid sequence wherein one, two, three or four, preferably one or two amino acid(s) from the amino acid sequence selected from the group consisting of SEQ ID NO. 3 to 14 has(have) been changed and selecting a polypeptide exhibiting a K_{D} to an antibody having an Ig light chain sequence according to SEQ ID 1 and an Ig heavy chain sequence according to SEQ ID NO. 2 of 150 x 10⁻⁶ M or less. The changes in the amino acid sequence may be amino acid exchanges, insertions, deletions, N-terminal truncations, or C-terminal truncations, or any combination of these changes, which may occur at one or two sites. Preferably, the change(s) is(are) an amino acid(s) exchange occurring at one or two sites. The amino acid exchanges may be conservative or non-conservative. In preferred embodiments the GD2 peptide mimotope comprises one or two conservative amino acid exchanges. "Non-conservative substitutions" or "non-conservative amino acid exchanges" are defined as exchanges of an amino acid by another amino acid listed in a different group of the six standard amino acid groups shown below:
(1) hydrophobic: M, A, V, L, or I;
(2) neutral hydrophilic: C, S, or T;
(3) acidic: D, or E;
(4) basic: N, Q, H, K, or R;
(5) residues that influence chain orientation: G, or P and
(6) aromatic: W, Y, or F.

Conservative exchanges are defined as exchanges of an amino acid by another amino acid listed within the same group of the six standard amino acid groups shown above.

The dissociation constant (K_{D}) of a polypeptide comprising a GD2 peptide mimotope can be calculated by any art known method it is, however, preferably calculated with the curve fitting program supplied by Biacore software. Preferably, the K_{D} of the polypeptide comprising a GD2 peptide mimotope 150 x 10⁻⁶ M, preferably below 140, 130, 120, 110, 100, 90, 80, 70, 60, 50, 40, 30, and most preferably below 20 x 10⁻⁶ M. The K_{D} is preferably determined at a temperature between 15 and 40°C, more preferably between 20 and 30°C and most preferably at about 25°C.

The polypeptides producible according to the present invention may be further modified as the polypeptides discussed above or may comprise any of the additional components and preferred components, i.e. N- and C-terminal cyclization moieties, immunogens, and/or uptake enhancing moieties.

Methods of producing such peptide mimotopes, i.e. comprising one, two, three or four amino acid exchanges, insertions, deletions N-terminal and/or C-terminal truncations, preferably one, two, three, or four mutations within the amino acid sequence as set out in SEQ ID NO. 3 to 14 are known in the art and comprise e.g. SPOT synthesis (Weise, A.A. et al. (2005) Analyt. Biochem. 342: 300-311) and silicon based array technologies as described by Jacobs J.W. and Fodor S.P. (1994) Trends Biotechnol. 12(1):19-26, US 5,143,854 and US 5,324,633.

In a further aspect the present invention relates to polynucleotide comprising a nucleotide sequence encoding a polypeptide of the present invention. The polynucleotides can be DNA, cDNA, synthetic DNA, RNA, and can be double-stranded or single-stranded.

In a further aspect the present invention relates to a vector comprising the polynucleotide of the present invention. The term "vector" refers to a means, including, for example, a protein or a polynucleotide or a mixture thereof, which is capable of being introduced or of introducing the polynucleotides of the invention into a cell. Certain vectors are particular suitable for the introduction of polynucleotides into only some specific cell types, while other vectors can be introduced into a variety of different cell types. The skilled artisan knows how to choose a particular vector depending on the cell type into which the polynucleotide or polypeptide is to be introduced.

In a preferred embodiment the vector of the present invention comprises plasmids; phagemids; phages, in particular M13 and derivatives thereof; cosmids; artificial chromosomes, in particular artificial mammalian chromosomes or artificial yeast chromosomes; knock-out or knock-in constructs; viruses, in particular adenovirus, vaccinia virus, attenuated vaccinia virus, canary pox virus, lentivirus (Chang and Gay, 2001), herpes virus, in particular Herpes simplex virus (HSV-1, Carlezon, *et al.,* 2000), baculovirus, retrovirus, adeno-associated-virus (AAV, Carter and Samulski. 2000), rhinovirus, human immune deficiency virus (HIV), filovirus, and engineered versions of above mentioned viruses (see, for example, Kobinger *et al.,* 2001); virosomes; "naked" DNA, liposomes; virus-like particles; and nucleic acid coated particles, in particular gold spheres. Particularly preferred are phages, plasmids and cosmids.

In a preferred embodiment of the present invention the vector is an expression vector, a gene targeting vector and/or a gene transfer vector.

In a further embodiment of the vector of the present invention the polynucleotide of the present invention encoding the polypeptide of the present invention is operatively linked to one or more expression control sequences, which allow expression in prokaryotic and/or eukaryotic host cells. The transcriptional/translational regulatory elements referred to above include, but are not limited to, inducible and non-inducible, constitutive, cell cycle regulated, metabolically regulated promoters, enhancers, operators, silencers, repressors and other elements that are known to those skilled in the art and that drive or otherwise regulate gene expression. Such regulatory elements include, but are not limited to, regulatory elements directing constitutive expression like, for example, promoters transcribed by RNA polymerase III like, e.g., promoters for the snRNA U6 or scRNA 7SK gene, the cytomegalovirus hCMV immediate early gene, the early or late promoters of SV40 adenovirus, viral promoter and activator sequences derived from, e.g., NBV, hepatits (HCV), herpes simplex virus (HSV), human papilloma virus (HPV), Ebstein-Barr virus (EBV), human T-cell leukaemia virus (HTLV), mouse mammary tumor virus (MMTV) or HIV; which allow inducible expression like, for example, CUP-1 promoter, the tetrepressor as employed, for example, in the tet-on or tet-off systems, the lac system, the trp system; regulatory elements directing cell cycle specific expression like, for example, cdc2, cdc25C or cyclin A promoter; or the TAC system, the TRC system, the major operator and promoter regions of phage A, the control regions of fd coat protein, the promoter for 3-phosphoglycerate kinase (PGK), the promoters of acid phosphatase, and the promoters of the yeast α- or a-mating factors. Particularly preferred promoters are the constitutive CMV immediate early gene promoter, the early or late SV 40 promoter, the polyhedrin promoter, retroviral LTRs, PGK promoter, élongation factor 1-α (EF1-α) and phosphoenolpyruvate carboxy kinase (PEPCK).

As used herein, "operatively linked" means incorporated into a genetic construct so that expression control sequences effectively control expression of a coding sequence of interest.

Another aspect of the present invention is a host cell genetically engineered with the polynucleotide(s) or the vector(s) of the present invention outlined above. The host cells that may be used for purposes of the invention include, but are not limited to, prokaryotic cells such as bacteria, preferably Gram-negative bacteria including *E. coli, Salmonella* spezies, in particular *Salmonella typhimurium, Lactococcus* species, e.g. *Lactococcus lactis,* and *Bacillus* species, e.g. *B. subtilis),* which can be transformed with, for example, recombinant bacteriophage DNA, plasmid DNA, or cosmid DNA expression vectors containing the polynucleotide molecules of the invention; simple eukaryotic cells like yeast (for example, *Saccharomyces* and *Pichia*), which can be transformed with, for example, recombinant yeast expression vectors containing the polynucleotide molecule of the invention; insect cell systems like, for example, *Spodoptera frugiperda* and *Trichoplusioa ni* cell lines, e.g. Sf9 or Hi5 cells, which can be infected with, for example, recombinant virus expression vectors (for example, baculovirus) containing the polynucleotide molecules of the invention; Xenopus oocytes, which can be injected with, for example, plasmids; plant cell systems, which can be infected with, for example, recombinant virus expression vectors (for example, cauliflower mosaic virus (CaMV) or tobacco mosaic virus (TMV)) or transformed with recombinant plasmid expression vectors (for example, Ti plasmid) containing a circularly permuted GFP polypeptide encoding nucleotide sequence; or mammalian cell systems (for example, COS, CHO, BHK, HEK293, VERO, HeLa, MDCK, Wi38, NSO and NIH 3T3 cells), which can be transformed with recombinant expression constructs containing, for example, promoters derived, for example, from the genome of mammalian cells (for example, the metallothionein promoter) from mammalian viruses (for example, the adenovirus late promoter and the vaccinia virus 7.5K promoter) or from bacterial cells (for example, the tetrepressor binding its employed in the tet-on and tet-off systems). Also useful as host cells are primary or secondary cells obtained directly from a mammal and transfected with a plasmid vector or infected with a viral vector. Depending on the host cell and the respective vector used to introduce the polynucleotide of the invention the polynucleotide can integrate, for example, into the chromosome or the mitochondrial DNA or can be maintained extrachromosomally like, for example, episomally or can be only transiently comprised in the cells. Preferred host cells are bacterial cells, in particular Gram negative bacteria, more preferably *Escherichia, Salmonella* or *Bacillus* species. To avoid pathogenic activity of the cells of the present invention, the cells of the present invention might be (rendered) replication incompetent in the patient body. Such replication incompetence might be achieved by radiation or by selection of strains not capable to grow in the environment of the patient due to an auxotrophy or the like.

If the polypeptide of the invention is expressed in a host cell it is particularly preferred that it is expressed as a fusion protein with an immunogen and/or a cellular uptake enhancing moiety as outlined above. Preferred immunogens in that respect are selected from the group consisting of T1 epitope of HIV gp 120; zymosan; heat shock proteins (HSP), in particular HSP 70; polycationic peptides, in particular poly-L-arginine; fibronectin; flagellin; cytokines with adjuvant activity, in particular GM-CSF, interleukin- (IL-)2, IL-6, IL-7, IL-11, IL-18, type I and II, interferons, in particular interferon-gamma, TNF-alpha; synthetic oligopeptides presented by MHCII.

It has been observed that the use of host cells expressing the polypeptides of the invention to actively vaccinate patients has elicited the highest anti GD2 humoral immune response and NK-cell activation. This is consistent with the fact that a non-replicating bacteria, in particular a non-replicating strain of *S. typhimurium* (SL7207) has been described as a suitable oral DNA vaccine delivery vehicle (Bolest E. *et al.* (2005) supra; Darji A. *et al.* (1997) Cell 91:765 and Lowrie D.B. (1998) Nat. Med. 4:147). Such attenuated bacteria appear to provide a "danger signal", enhance antigen presentation and promote a TH₁ cellular immune response.

In a further aspect the present invention relates to a pharmaceutical composition comprising a polypeptide of the invention, a polynucleotide of the invention, a vector of the invention or a host cell of the invention and a pharmaceutically acceptable adjuvant.

It is particularly preferred that the pharmaceutical composition comprising a polypeptide of the invention, a polynucleotide of the invention, a vector of the invention or a host cell of the invention can be used in the form of systemically administered medicaments. These include parenterals, which comprise among others injectables and infusions. Injectables are formulated either in the form of ampoules or as so called ready-for-use injectables, e.g. ready-to-use syringes or single-use syringes and aside from this in puncturable flasks for multiple withdrawal. The administration of injectables can be in the form of subcutaneous (s.c.), intramuscular (i.m.), intravenous (i.v.) or intracutaneous (i.e.) application. In particular it is possible to produce the respectively suitable injection formulations as a suspension of crystals, solutions, nanoparticular or a colloid dispersed systems like, e.g. hydrosols.

Injectable formulations can further be produced as concentrates, which can be dissolved or dispersed with aqueous isotonic diluents. The infusion can also be prepared in form of isotonic solutions, fatty emulsions, liposomal formulations and micro emulsions. Similar to injectables infusion formulations can also be prepared in the form of concentrates for dilution. Injectable formulations can also be applied in the form of permanent infusions both in in-patient and ambulant therapy, e.g. by way of mini-pumps.

It is possible to add to parental drug formulations, for example, albumin, plasma, expander, surface-active substances, organic diluents, pH-influencing substances, complexing substances or polymeric substances, in particular as substances to influence the adsorption of the polypeptides of the invention, the polynucleotides of the invention, the vectors of the invention or the host cells of the invention to proteins or polymers or they can also be added with the aim to reduce the adsorption of the polypeptides of the invention, the polynucleotides of the invention, the vectors of the invention or the host cells of the invention to materials like injection instruments or packaging-materials, for example, plastic or glass.

The polypeptides of the invention, the polynucleotides of the invention, the vectors of the invention or the host cells of the invention can be bound to microcarriers or nanoparticles in parenterals like, for example, to finely dispersed particles based on poly(meth)acrylates, polylactates, polyglycolates, polyamino acids or polyether urethanes. Parenteral formulations can also be modified as depot preparations, e.g. based on the "multiple unit principle", if the polypeptides of the invention, the polynucleotides of the invention, the vectors of the invention or the host cells of the invention are introduced in finely dispersed, dispersed and suspended form, respectively, or as a suspension of crystals in the medicament or based on the "single unit principle" if the polypeptides of the invention, the polynucleotides of the invention, the vectors of the invention or the host cells of the invention is enclosed in a formulation, e.g. in a tablet or a rod which is subsequently implanted. These implants or depot medicaments in single unit and multiple unit formulations often consist out of so called biodegradable polymers like e.g. polyesters of lactic and glycolic acid, polyether urethanes, polyamino acids, poly(meth)acrylates or polysaccharides.

Adjuvants and carriers added during the production of the pharmaceutical compositions of the present invention formulated as parenterals are preferably aqua sterilisata (sterilized water), pH value influencing substances like, e.g. organic or inorganic acids or bases as well as salts thereof, buffering substances for adjusting pH values, substances for isotonization like e.g. sodium chloride, sodium hydrogen carbonate, glucose and fructose, tensides and surfactants, respectively, and emulsifiers like, e.g. partial esters of fatty acids of polyoxyethylene sorbitans (for example, Tween^{®}) or, e.g. fatty acid esters of polyoxyethylenes (for example, Cremophor^{®}), fatty oils like, e.g. peanut oil, soybean oil or castor oil, synthetic esters of fatty acids like, e.g. ethyl oleate, isopropyl myristate and neutral oil (for example, Miglyol^{®}) as well as polymeric adjuvants like, e.g. gelatine, dextran, polyvinylpyrrolidone, additives which increase the solubility of organic solvents like, e.g. propylene glycol, ethanol, N,N-dimethylacetamide, propylene glycol or complex forming substances like, e.g. citrate and urea, preservatives like, e.g. benzoic acid hydroxypropyl ester and methyl ester, benzyl alcohol, antioxidants like e.g. sodium sulfite and stabilizers like e.g. EDTA.

When formulating the pharmaceutical compositions of the present invention as suspensions in a preferred embodiment thickening agents to prevent the setting of the polypeptides of the invention, the polynucleotides of the invention, the vectors of the invention or the host cells of the invention or, tensides and polyelectrolytes to assure the resuspendability of sediments and/or complex forming agents like, for example, EDTA are added. It is also possible to achieve complexes of the active ingredient with various polymers. Examples of such polymers are polyethylene glycol, polystyrol, carboxymethyl cellulose, Pluronics^{®} or polyethylene glycol sorbit fatty acid ester. The polypeptides of the invention, the polynucleotides of the invention, the vectors of the invention or the host cells of the invention can also be incorporated in liquid formulations in the form of inclusion compounds e.g. with cyclodextrins. In particular embodiments dispersing agents can be added as further adjuvants. For the production of lyophilisates scaffolding agents like mannite, dextran, saccharose, human albumin, lactose, PVP or varieties of gelatine can be used.

In a preferred embodiment the pharmaceutical composition of the present invention, which may or may not already comprise an immunogen fused to the GD2 peptide mimotope may further comprise and immunogen. Preferred immunogens are selected from the group consisting of unmethylated DNA, in particular unmethylated DNA comprising CpG dinucleotides (CpG motif), in particular CpG ODN with phosphorothioate (PTO) backbone (CpG PTO ODN) or phosphodiester (PO) backbone (CpG PO ODN); bacterial products from the outer membrane of Gram-negative bacteria, in particular monophosphoryl lipid A (MPLA), lipopolysaccharides (LPS), muramyl dipeptides and derivatives thereof; synthetic lipopeptide derivatives, in particular Pam₃Cys; lipoarabinomannan; peptidoglycan; zymosan; heat shock proteins (HSP), in particular HSP 70; dsRNA and synthetic derivatives thereof, in particular Poly I:poly C; polycationic peptides, in particular poly-L-arginine; taxol; fibronectin; flagellin; imidazoquinoline; cytokines with adjuvant activity, in particular GM-CSF, interleukin- (IL-)2, IL-6, IL-7, IL-18, type I and II, interferons, in particular interferon-gamma, TNF-alpha; 25-dihydroxyvitamin D3 (calcitriol); synthetic oligopeptides, in particular MHCII-presented peptides; gel-like precipitates of aluminum hydroxide (alum).

In a further aspect the present invention relates to the use of a polypeptide of the invention, a polynucleotide of the invention, a vector of the invention or a host cell of the invention for the production of a medicament for the treatment of tumors of neuroectodermal origin. In a preferred embodiment the tumor of neuroectodermal origin is selected from melanoma, neuroblastoma, glioma, soft tissue sarcoma and small cell carcinoma of the lung.

### BRIEF DESCRIPTION OF THE FIGURES

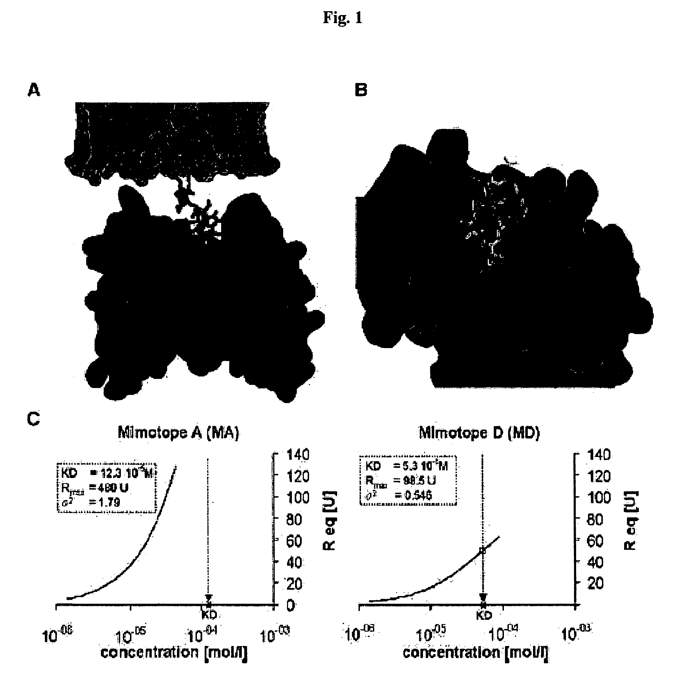

- **Fig. 1:**: Docking of ganglioside GD2 and GD2 peptide mimotopes to Mab ch14.18.
A) Computational model of the best docking result representing the contact between nominal antigen GD2 and the paratope of the modelled anti-GD2 Mab ch14.18. GD2 is anchored in the putative neuroblastoma cell membrane with its two fatty acid chains.
B) Circular peptides MA (light grey) and MD (dark grey) were docked into the binding site of ch14.18 as described below. Bonds of the peptide backbone are represented by thick lines, bonds of side chains are indicated by thin lines. The calculated free energies of circular MA and MD binding were -41.23 kJ/mol and -48.06 kJ/mol, respectively.
C) Binding affinities of the GD2 mimicking peptides to chimeric anti-GD2 Mab ch14.18. The binding affinity was measured using the Biacore system for the analysis of protein interactions. Results represent the response in equilibrium (Req) depending on the concentration of Mab ch14.18 [mol/l]. The dissociation constant (K_{D}) and the maximum response (Rₘₐₓ) were calculated with the curve fitting program, supplied by
Biacore software.

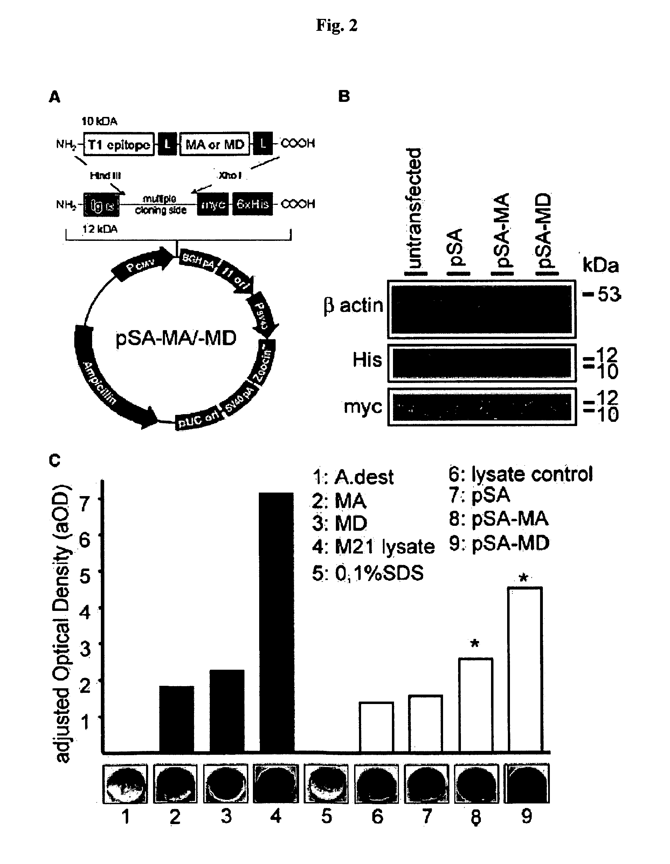

- **Fig. 2:**: Schematic, protein expression and GD2 mimicry of DNA vaccines encoding GD2 peptide mimotopes. A) Schematics illustrate the generated DNA minigene vaccines (pSA-MA, pSA-MD) which encode the GD2 mimicking peptide mimotopes, i.e. mimotope A and D (MA, MD). The pSA-MA and pSA-MD plasmids are based on pSecTag2 A (pSA) including a kappa leader sequence (Ig κ), a T-cell helper epitope from HIV-1 gp120 (T1) and two flexible glycin-serin linkers.
B) Minigene expression of pSA-MA and pSA-MD was demonstrated in CHO-1 cells by Western blot using anti-His and anti-Myc tag antibodies.
C) GD2 mimicry was analyzed following transfection of CHO-1 cells (1 x 10⁶) with GD2 peptide mimotope DNA vaccines by dot blot experiments using anti-GD2 Mab 14G2a. Signals were compared to synthetic GD2 mimotope peptides (4 µg) and a lysate of GD2 positive M21 cells (1.25 x 10⁵), both used as positive controls. Peptide dot blot buffer (1) and cell lysis buffer (5) were used as negative controls. Results represent adjusted optical densities measurements (aOD) (top) and photographs of representative dots (bottom) (* p ≤ 0.05, Student-t-Test).

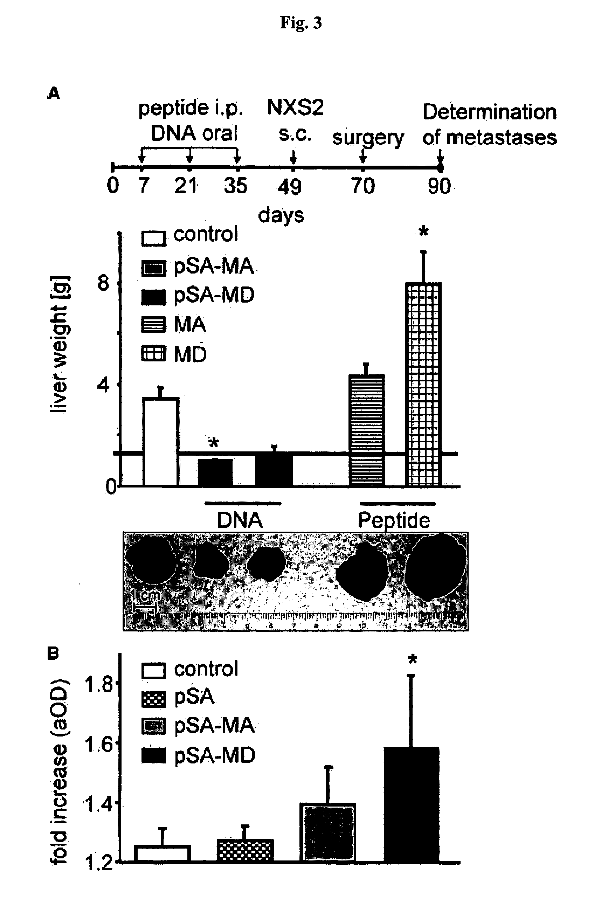

- **Fig. 3:**: Effects of DNA versus peptide vaccination with plasmids encoding for GD2 mimotopes and synthetic GD2 peptide mimotopes in a syngeneic neuroblastoma model. Mice were immunized either by oral gavage of pSA-MA, pSA-MD transfected attenuated *Salmonella typhimurium* or by i.p. injection of KLH conjugated synthetic GD2 peptide mimotopes (MA, MD) adsorbed to Al(OH)₃. All mice (control n=6, experimental groups n=8) were challenged by s.c. injection of NXS2 neuroblastoma.
A) Spontaneous liver metastasis was determined by measurements of liver weights of fresh specimens. Results represent mean ± S.E.M. (* p ≤ 0.05). The horizontal line indicates the liver weight of mice without metastases (1.2 g). Photographs of one representative liver specimen from each group of mice are shown.
B) Blood samples taken one week before the 1^{st} and one week after the last immunization were analyzed by GD2 ELISA. Results (mean ± S.E.M.) of the anti-GD2 serum response following DNA vaccination represent aOD values. s.c. = subcutaneous, i.p. intra peritoneal (* p ≤ 0.05, Student-t-Test).

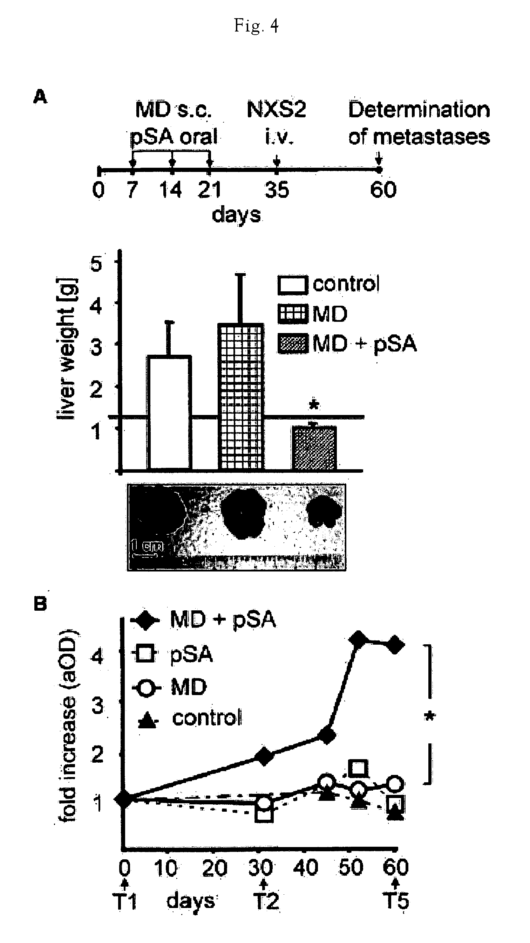

- **Fig. 4:**: Effect of co-administration of oral DNA adjuvants in GD2 peptide-mimotope vaccinated A/J mice. Mice were immunized by s.c. injection of KLH conjugated synthetic GD2 peptide mimotopes (MD) adsorbed to Al(OH)3 with and without co-administration of oral DNA (pSA empty vector delivered by attenuated *Salmonella typhimurim*)(*pSA*). All mice (control n=6, experimental groups n=8) were challenged by i.v. injection of NXS2 neuroblastoma.
A) Spontaneous liver metastasis was determined by measurements of liver weights of fresh specimens. Results represent mean ± S.E.M. (*: p ≤ 0.05). The horizontal line indicates normal liver weights of mice without metastases (1.2 g). Representative liver photographs from each group of mice are shown.
B) The course of anti-GD2 serum responses in peptide-vaccinated mice with and without oral application of oral DNA (pSA) is shown. Samples were taken on time points indicated by an arrow. Results (mean) are illustrated as x fold increase in contrast to the pre-immunization anti-GD2 serum level (T1) (Student-t-Test, *: p ≤ 0.05). T = time point, s.c. = subcutaneous, i.p. intra peritoneal).

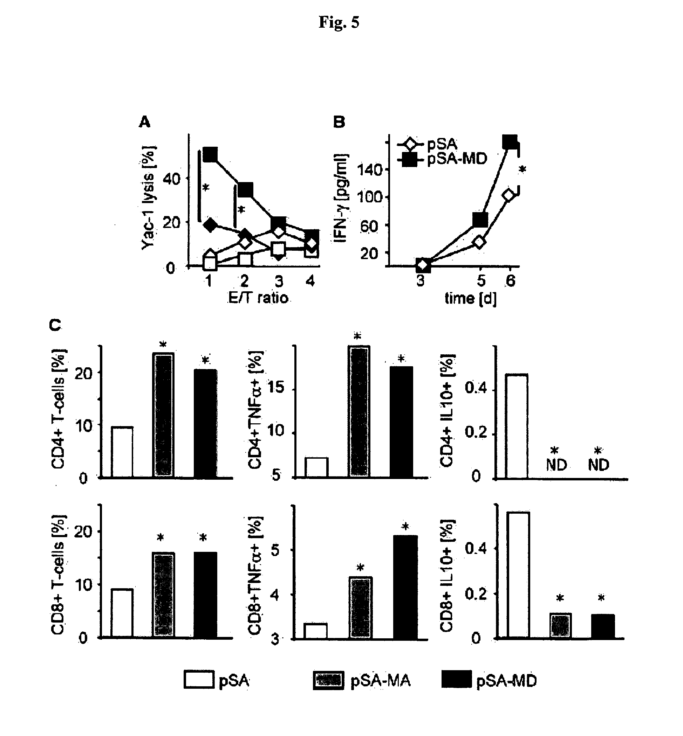

- **Fig. 5:**: Immune response following vaccination with DNA vaccines encoding GD2 peptide mimotopes. Mice were immunized with the appropriate DNA vaccines and then subjected to the analysis of NK cell activation, release of IFN-γ and intracellular cytokine expression of T-cell subpopulations. All mice (control n=6, experimental groups n=8) were challenged with NXS2 cells
A) NK cell activation was determined with pooled splenocytes of mice immunized with pSA-MD (square) and pSA (diamond) control at varying effector to target cell ratios (1=100:1, 2=50:1, 3=25:1, 4=12.5:1) 7 days after co-culture in the presence (closed symbols) and absence (open symbols) of irradiated NXS2 cells (* Mann-Whitney; p≤0.05).
B) IFN-γ-secretion from splenocytes of mice immunized with GD2 peptide mimotope DNA vaccine pSA-MD was determined over time in the presence of irradiated NXS2 cells (χ² Test; *: p ≤0.05).
C) Intracellular cytokine expression was determined in T-cell subpopulations. TNF-α and IL10 expression was analyzed in CD4⁺ and CD8⁺ T cells of vaccinated mice. The percentages of CD4⁺ or CD8⁺ T-cells as well as the percentages of TNF-α and IL10 producing CD3⁺CD4⁺ or CD3⁺CD8⁺ T-cells were calculated relative to the total number of CD3⁺ T-cells (χ² Test; * p ≤ 0.05 ; ND = non detectable).

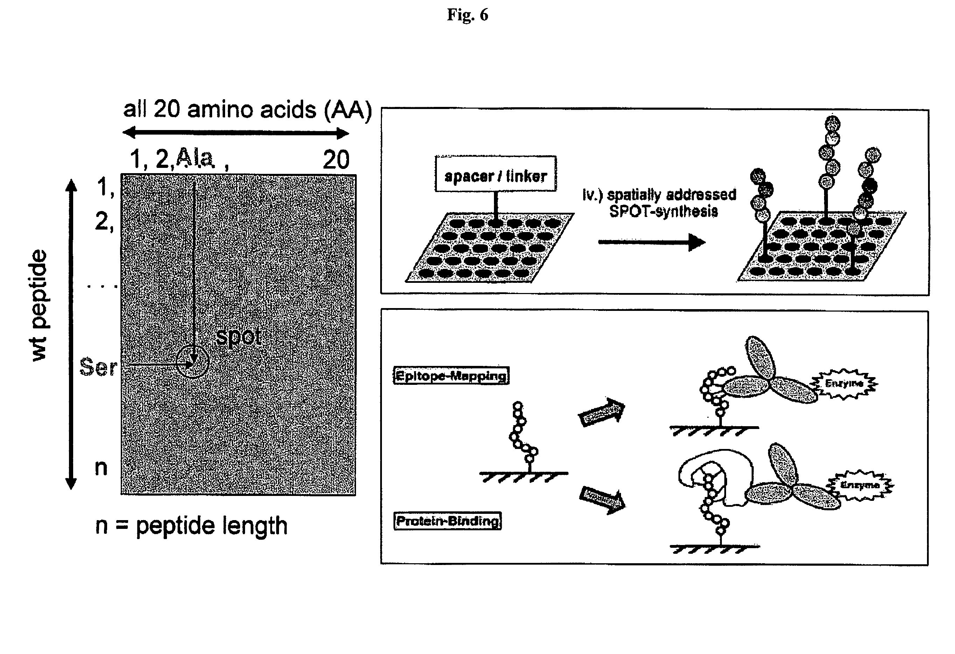

- **Fig. 6:**: Schematic representation of the optimization of peptide binding by SPOT-synthesis. On the left side of the left panel the wild type (wt) sequence of the respective peptide to be mutated is indicated, while on the top of the left panel all 20 amino acids are indicated. In each lane the respective wt amino acid is replaced by all 19 other amino acids. One sequence in each lane is wt sequence and all others are single point mutants. Accordingly, for a wild type peptide of 20 amino acids length 400 different 20 mers are arranged in a two-dimensional grid out of which 380 are different single point mutants and 20 are wt.

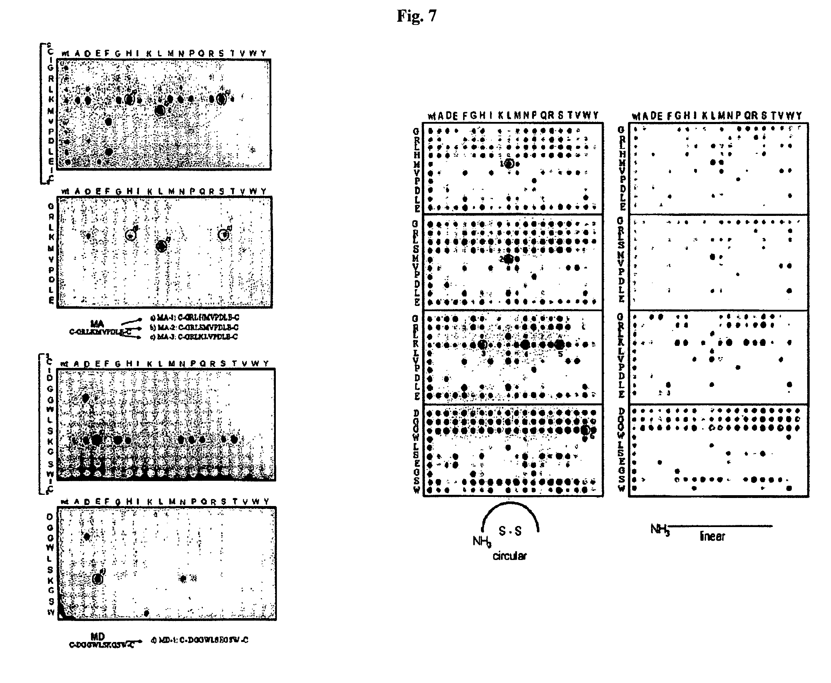

- **Fig. 7:**: Optimization of GD2 mimotopes by peptide mutation analysis. The four left peptide grids depict a first round of mutations of the wt sequences of the GD2 mimotopes with the sequences GRLKMVPDLE (SEQ ID NO. 3) (the two upper grids) and DGGWLSKGSW (SEQ ID NO. 10) (the two lower grids). The upper of the two pairs of grids depicts binding using circularized peptides.
The right panel of eight peptide grids depicts a secondary mutational analysis on the basis of the optimized GD2 mimotopes with the sequences GRLHMVPDLE (SEQ ID NO. 4), GRLSMVPDLE (SEQ ID NO. 5), and GRLKLVPDLE (SEQ ID NO. 6) and DGGWLSEGSW (SEQ ID NO. 11). The left four grids depict binding using circularized peptides.

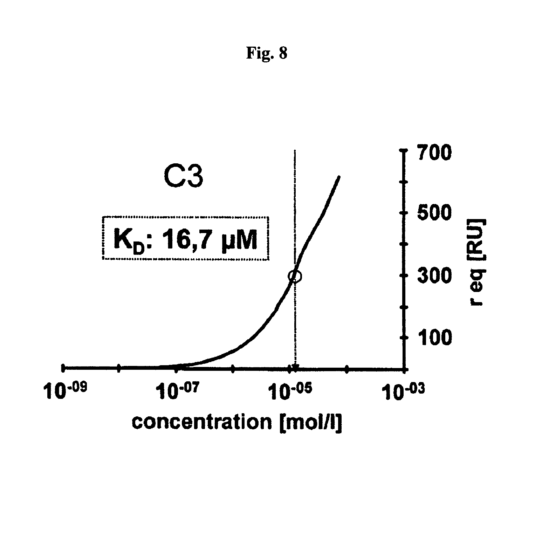

- **Fig. 8:**: Dissociation constants (K_{D}). *In vitro* dissociation constants (K_{D}) of optimized GD2 mimotope C3 CGRLHLVPDLEC (SEQ ID NO. 17). The dissociation constant is measured by surface plasmon resonance (Biacore).

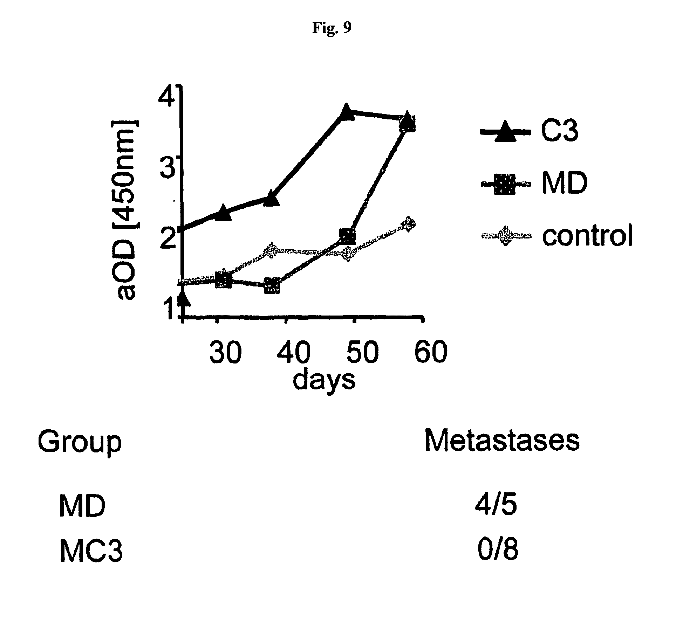

- **Fig. 9:**: Anti-GD2 serum response. *In vivo* anti-GD2 serum response measured for optimized C3 mimotope, MD mimotope and control. Lower panel indicates number of metastasis in MD treated and C3 treated group.

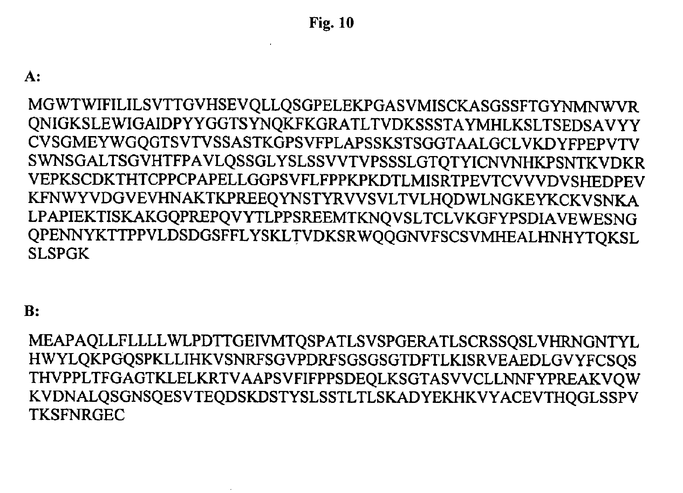

- **Fig. 10:**: Amino acid sequence of the anti-GD2 specific monoclonal antibody ch14.18. Panel A depicts the heavy chain pre-peptide. Panel B depicts the light chain pre-peptide.

### EXAMPLES

**Computer modeling**: All template structures were identified for the generation of a model for the variable domain of ch14.18 Mab using the PSI-BLAST program (Altschul SF, et al. (1997) Nucleic Acids Res. 25: 3389-402). The amino acid sequence was that described previously (Bolesta E, et al. (2005) Cancer Res. 65: 3410-8). The heavy and light chains (VH and VL) were considered separately. Protein Data Bank (PDB) (Berman HM, et al. (2000) Nucleic Acids Res 28: 235-42) structure 1f3d was found to be the best template structure for the VH and 1jgu for the VL, resulting in 71% and 96% sequence identity, respectively. In contrast, the 1jp5 template previously used (Bolesta E, *et al.* (2005) *supra*) shows an identity of only 67%. Homology modelling was performed with the aid of the Swiss-PdbViewer (Guex N, Peitsch MC. (1997) Electrophoresis 18: 2714-23).

When necessary, loop structures were extracted from the LIP database (Michalsky E, et al. (2003) Protein Eng. 16: 979-85). In a second step the binding of GD2 to Mab ch14.18 was analyzed. The planar structure of GD2 was obtained from (Ragupathi G. (1996) Cancer Immunol Immunother 43: 152-7). Three-dimensional conformers were generated using the software Catalyst (Accelrys Software Inc.). These were docked onto the antibody model with aid of GRAMM (38). Models for the linear and circular peptides MA (C-GRLKMVPDLE-C, SEQ ID NO. 15), MD (C-DGGWLSKGSW-C, SEQ ID NO. 16) and C3 (C-GRLHLVPDLE-C, SEQ ID NO 17) were built using the Swiss-Pdb Viewer. We calculated the ranking according to GOLD score and the binding free energy of the interaction between the peptides (MA, MD) and the nominal GD2 antigen with Mab ch14.18 (Verdonk ML, et al. (2005) J Med Chem 48: 6504-15).

### Peptide synthesis and minigene construction of GD2 mimicking DNA vaccines:

Minigenes encoding for GD2 mimicking peptides (Table 1), flanked by two (Gly₃Ser)₄ linkers and the T1 epitope (Fig. 2 A) were created by overlapping PCR as previously described (Huebener N, et al. (2003) Cancer Lett 2003;197: 211-7). PCR fragments were isolated and cloned into pGEM^{®} T easy vector following the manufacturers' guidelines (Promega, Germany) and subjected to molecular sequencing. Correct inserts were subcloned into pSA using restriction enzymes *Hind*III/*Xho*I*.* The decapeptides MA, MD and C3 were synthesized, cyclized and conjugated to KLH by JPT GmbH (Berlin, Germany).

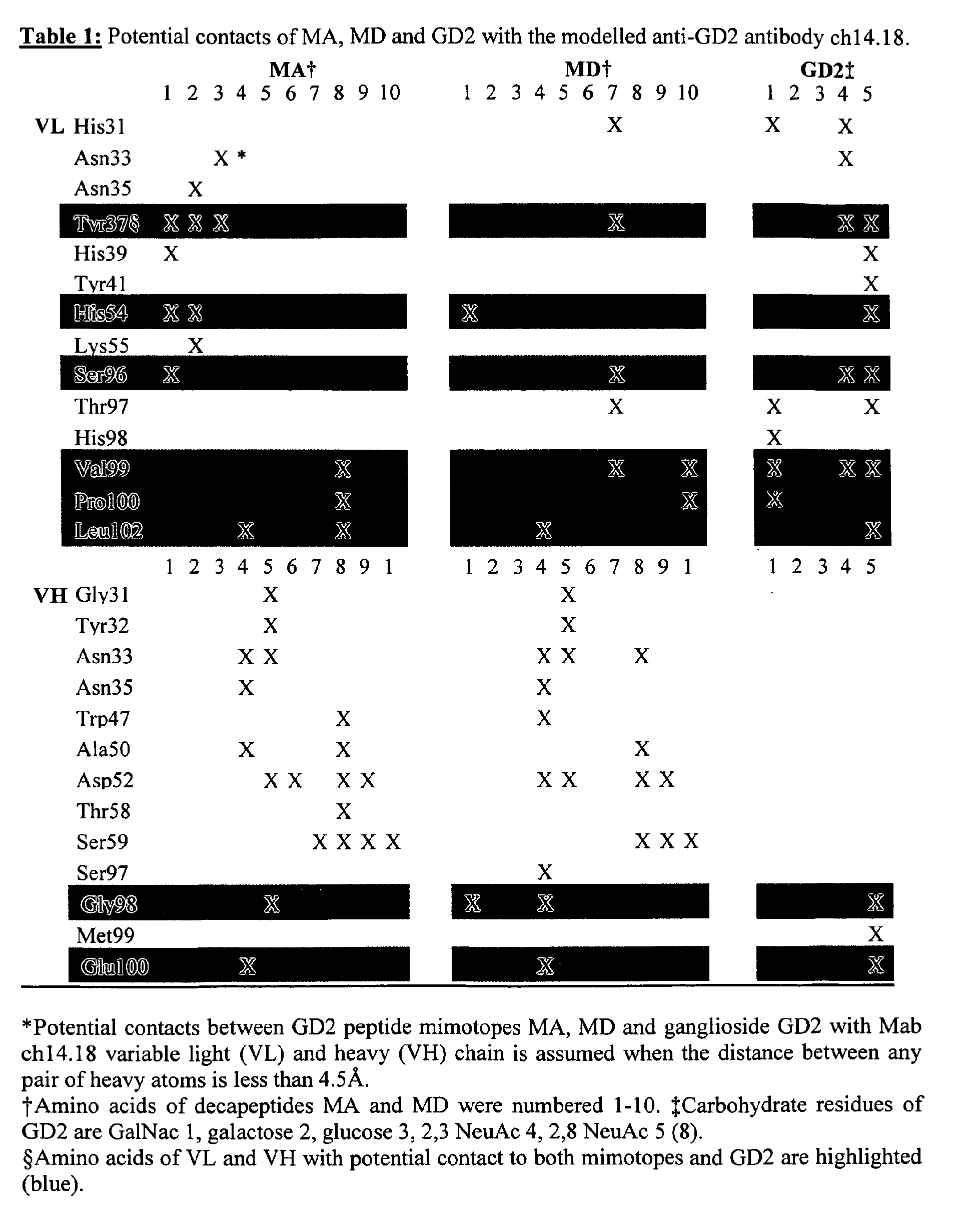

**Protein expression analysis:** The expression of minigene peptides was tested after transient transfection of the DNA vaccines (pSA-MA, pSA-MD and pSA-C3) into CHO-1 cells using the Fugene 6^{®} transfection reagent (Roche, Germany). After 72 hours cells were lysed (0.1% SDS in PBS, pH 7.4, 4°C, 30 min) and centrifuged (10,000 g, 4°C, 45 min). Lysates were separated by SDS-PAGE using the Novex system (Invitrogen, Germany), and blots were incubated with anti-myc- and and anti-His antibodies (Invitrogen, Germany) according to the manufacturers protocol. POD-labelled anti-mouse IgG antibody (Sigma, Germany) was added (1:1000, Tween20 0.1%, 5% milk, PBS, pH 7.4). The membrane was washed (3x, 15 min, RT) and signals were analyzed with the ECL™ Detection Reagents (Amersham, Germany).

**Analysis of GD2 mimicry:** Mimicry analysis was accomplished following lysis of 1x10⁶ transfected cells in 100 µl lysis buffer (SDS 0.1% in PBS, pH 7.4). After centrifugation (10,000 g, 4°C, 45 min), 100 µl of the supernatant was dotted onto a nitrocellulose membrane by vacublotting using the Delrin dot blotting apparatus (Schleicher and Schuell, Germany). The membrane was blocked (5% milk, 0.1% Tween20, PBS, pH 7.4) and incubated (1h, RT) with anti-GD2 antibody (14G2a, Pharmingen, Germany) at 1 µg/ml PBS (pH 7.4, 4°C). After washing, the membrane was incubated with POD-labelled anti-mouse IgG Ab (1:1000, 8µl, 1h, RT) (Sigma, Germany). Signals were analyzed with the AEC developing kit (Vector Laboratories, Germany). Signal intensity was measured with the GS-800 densitometer (Bio-Rad Laboratories GmbH, Germany). The adjusted optical density (aOD) of one dot was calculated as follows: mean OD inside the volume area multiplied with the dot area [mm²] minus aOD of the membrane background (GS-800 software from Bio-Rad Laboratories GmbH, Germany). Each dot represents the aOD of lysate prepared from 1 x 10⁶ CHO-1 cells.

**Surface plasmon resonance (SPR) measurement:** The affinities of the peptides to Mab ch14.18 were determined by SPR measurement in a BiacoreX-system (Uppsala, Sweden) in HBS-buffer (10 mM 2-[4-(2-hydroxyl)-1-piperazino]ethane-sulfonic acid (HEPES), pH 7.4, 150 mM NaCl, 3 mM ethylenediamine-*N,N,N',N*'-teraacetic acid (EDTA), 0.005% Surfactant P20). Each cyclized peptide CDGGWLSKGSWC (peptide MD) CGRLKMVPDLEC (peptide MA) and CGRLHLVPDLEC (peptide C3) was immobilized on a CM5-sensor-chip (measurement cell) via the amine-coupling-method as described by the producer. Irrelevant peptide (SATPWDLKTSL, SEQ ID NO. 18) was attached with the same method and used in the reference-cell. Affinity of chimeric anti-GD2 Mab ch14.18 to mimotope peptides was determined using a dilution-series of Mab ch14.18 from 90 µM - 1.4 µM (MD), and 46.6 µM-1.5 µM (MA). All binding-experiments were executed at 25°C with a flow rate of 5 µl/min. Data were analyzed using the equilibrium-method of the program BIAevaluation 3.0.

**Cell culture and mice:** The NXS2 murine neuroblastoma model was previously described (Lode H.N. et al. (1997) Journal of the National Cancer Institute 89: 1586-94). Syngeneic female A/J mice (8-10 weeks old) were obtained (Harlan Winkelmann, Borchen, Germany) and housed according to the German guide for the care and use of laboratory animals, i.e. 'Tierschutzgesetz'. Once liver metastases were detectable in control groups, mice were sacrificed and splenocytes were isolated and cultured in RPMI (10% FCS, 100 µg/ml penicillin-streptomycin, 50 µM β-Mercaptoethanol, 100 IU/ml IL-2) in the presence or absence of irradiated (50 Gy, 10 min) NXS2 neuroblastoma cells (1:100) for seven days.

**Immunization procedure and analysis of the tumor growth and metastasis:** First , DNA vaccines were delivered to A/J mice (n = 6-8) every 2 weeks (4x) by oral gavage of 1×10⁸ *Salmonella typhimurium* SL7207 carrying the minigene-vector constructs or empty vector controls. Peptide vaccines were administered to mice by i.p. injection with KLH-conjugated mimotopes A and D (MA, MD) (10 µg/100 µl PBS) adsorbed to 100 µl Al(OH)₃ (Imject Alumn, Pierce) at identical time points (4x) (24). Second, peptide vaccination was applied as described above with or without the co-administration of DNA adjuvants (pSA empty vector delivered by attenuated *Salmonella typhimurim*). Blood samples were taken at indicated time points: T1: 7 days before 1^{st} immunization, T2 - T5: weekly after the immunization period. The induction of spontaneous liver metastases using 2×10⁶ NXS2 cells s.c. or 1x10⁵ NXS2 cells i.v. was done as previously described (40). Spontaneous liver metastases were analyzed by determination of liver weight.

**Determination of the anti-GD2 serum response:** Disialoganglioside GD2 (Sigma, Germany) was coated onto standard ELISA plates (96 well, flat bottom, Greiner, Germany) at 50 ng/50 µl methanol/well. Plates were blocked with 1% BSA/PBS (pH 7.4) (2 h, 37°C). Serum incubation was accomplished after 4 serial 1:2 dilutions (2h, 37°C). After washing (3x, PBS, 0.1% BSA, pH 7.4), the secondary anti-mouse IgG peroxidase conjugate (Sigma, Steinheim, Germany) antibody was added (1:10,000, 2 h, 37°C). Substrate development was induced after washing (3x, PBS, 0.1% BSA, pH 7.4) using substrate reagent pack DY999 (R&D Systems, Minneapolis, USA) according to the manufacturers guidelines. The reaction was stopped with 4 N H₂SO₄. OD was determined in a standard ELISA reader at 450 nm. The adjusted OD (aOD) was calculated as follows: aOD = OD of T1-5 / OD of T1.

**Cytotoxicity assays**: Lysis of NXS2- and YAC-1-target cells was determined in a standard ⁵¹Cr-release assay (Zeng Y, et al. (2005) Mol Immunol. 42: 1311-9). Per cent lysis was calculated as follows: (sample release - spontaneous release) / (maxium release - spontaneous release) x 100.

**IFN-γ secretion assay:** IFN-γ secretion was determined using the IFN-γ ELISA kit (BD OptEIA SET, BD Pharmingen). Supernatants (300 µl) of splenocytes co-cultured in the presence and absence of irradiated NXS2 cells were collected at several time points and stored at -80°C. On the day of measurement 50 µl samples of the supernatant were analyzed in duplicates.

**Flow cytometry:** The percentage of TNF-α and IL10 producing CD3⁺CD4⁺ and CD3⁺CD8⁺T-cells was determined by FACS analysis. Freshly isolated splenocytes were resuspended in DMEM (10% FCS) and treated for 3 hours with a cocktail of monensin (2µM), ionomycin (1 µg/ml) and phorbol 12 myristate 13-acetat (PMA, 50 ng/ml) (Sigma-Aldrich, Taufkirchen, Germany). Cells were fixed in PBS containing 1% paraformaldehyd (PFA) (Sigma-Aldrich, Taufkirchen, Germany) (30 min, 4°C, dark). Cells were washed (1% BSA, 0.1% sodium azide, PBS, pH 7.4) and incubated (4°, 30 min) with Abs directed against lymphocyte surface molecules (1:100): anti-CD3-PerCP, anti-CD4-FITC or anti-CD8a-FITC, anti-CD25-PE (BD Bioscience, Heidelberg, Germany). Cells were washed and permeabilised (PBS, pH 7.4, 0.1% saponin) (Sigma-Aldrich, Taufkirchen, Germany). Intracellular cytokines were determined with anti-TNF-□PE and anti-IL-10-PE Abs (1:200, 20 min, 4°C, dark) (BD Bioscience, Heidelberg, Germany). Samples were analyzed using a FACS Calibur (Becton Dickinson, Germany) equipped with "Cell Quest".

**Statistics:** Non-parametric data were analyzed by the Kruskal-Wallis-Test and the Mann-Whitney U-Test. Parametric data were analyzed with the Student-t-Test. The data from pooled splenocytes, investigated by cytotoxicity assays and flow cytometry, were analyzed by the □2 Test. Differences were considered significant at p < 0.05.

### Complete Substitution analysis of peptides:

a) *SPOT synthesis:* Synthesis of cellulose-membrane-bound arrays was done according to: H. Wenschuh, H. Gausepohl, L. Germeroth, M. Ulbricht, H. Matuschewski, A. Kramer, R. Volkmer-Engert, N. Heine, T. Ast, D. Scharn, J. Schneider-Mergener in Combinatorial Chemistry, A Practical Approach. (Ed.: H. Fenniri), Oxford University Press, Oxford (2000) 95-116. Purity of SPOT synthesized peptides is generally in the range of 40 - 85% which is adequate for screening assays. A peptide array presenting a complete substitution analysis is characterized by the exchange of all peptide sequence residues, one at a time, by each of the canonical amino acids. Additionally for a control the unmodified peptide sequence (wt) is synthesized several times at the array.
b) *Antibody binding assay:* The cellulose-membrane-bound complete peptide substitution arrays were washed with DMF, ethanol, and three times with TBS buffer (13.7 mM NaCl, 0.27 mM KCl, 5 mM Tris (tris(hydroxymethyl)amino-methane, pH 8.0)) for 10 min each. Membranes were incubated for 3 h with blocking buffer (for 50 mL blocking buffer: 5 mL blocking buffer (Sigma-Aldrich, Steinheim, Germany), 2.5 g sucrose, 5 mL 10 × TBS buffer (137 mM NaCl, 2.7 mM KCl, 50 mM Tris, pH 8.0), filled up to 50 mL with water). Binding of the peptide substitution variants to the anti-GD2 Mab ch14.18 was done as follows. The membrane bound arrays were incubated with the anti-GD2 mab ch14.18 (1 µg/ml), dissolved in the same blocking buffer at 4°C for 14 h. The membrane was then washed four times with TBS buffer and then incubated with the secondary anti-mouse IgG peroxidase conjugate (Sigma, Steinheim, Germany) antibody. After washing the membrane four times with TBS buffer the binding was visualized using a chemoluminescense substrate (Uptilight HRP, Uptima, Interchim, Montluon, France) and the Lumi-Imager™ (Roche Diagnostics).
*c) Measurement of spot signal intensities:* Analysis and quantification of spot signal intensities were executed with the software Genespotter (MicroDis-covery GmbH, Berlin, Germany). Genespotter has a fully automatic grid finding routine resulting in reproducible signal intensities. The spot signal is calculated from a circular region around the spot center detected on the image. The background signal for each spot is determined with a safety margin to this circular region.

## Claims

1. Polypeptide comprising a GD2 peptide mimotope having the amino acid sequence
GRLX₁X₂VPDLE,
wherein
X₁ is K, H, S, or N
X₂ is M, I or L
or
DGX₃WLSX₄GSW
wherein
X₃ is G, W, E, or D
X₄ is E, K, or N
or a variant thereof, wherein the variant comprises one or two further amino acid substitutions and exhibits a K_{D} to an antibody comprising the mature Ig light chain sequence derived from the light chain pre-protein according to SEQ ID 1 and the mature Ig heavy chain sequence derived from the heavy chain pre-protein according to SEQ ID NO. 2 of 150 x 10⁻⁶ M or less.

2. Polypeptide according to claim 1, having an amino acid sequence selected from the group consisting of SEQ ID NO. 3 to 14 or a variant thereof, wherein the variant comprises one or two further amino acid substitutions and exhibits a K_{D} t to an antibody comprising the mature Ig light chain sequence derived from the light chain pre-protein according to SEQ ID 1 and the mature Ig heavy chain sequence derived from the heavy chain pre-protein according to SEQ ID NO. 2 of 150 x 10⁻⁶ M or less.

3. Polypeptide according to claims 1 or 2 comprising at least one cyclization moiety N- and C-terminal of the GD2 peptide mimotope.

4. Polypeptide according to claim 3, wherein the cyclization moiety is selected from the group consisting of Cys, or Homo-Cys or the cyclisation moity is selected from a combination of the following pairs (one at the N-terminus the other at the C-terminus or vice versa): jodo-, chloro- or bromoacetic acid and Cys, jodo-, chloro- or bromacetic acid and Homo-Cys, 2-maleimidoacetic acid and Cys, 2-maleimidoacetic acid and Homo-Cys, 3-maleimidopropionic acid and Cys, 3-maleimidopropionic acid and Homo-Cys, 6-maleimidocaproic acid and Cys, 6-maleimidocaproic acid and Homo-Cys or combinations of the pairs: C-terminal Glu and any N-terminal canonical amino acid, C-terminal Asp and any N-terminal canonical amino acid.

5. Polypeptide according to claims 3 or 4, wherein the N- and C-terminal cyclization moieties are directly linked to the N- and C-terminus of the GD2 peptide mimotope.

6. Polypeptide according to claims 1 to 5 further comprising an immunogen.

7. Polypeptide according to claim 6, wherein the immunogen is selected from the group consisting of T1 epitope of HIV gp 120; zymosan; heat shock proteins (HSP), in particular HSP 70; polycationic peptides, in particular poly-L-arginine; fibronectin; flagellin; cytokines with adjuvant activity, in particular GM-CSF, interleukin- (IL-)2, IL-6, IL-7, IL-11, IL-18, type I and II, interferons, in particular interferon-gamma, TNF-alpha; synthetic oligopeptides presented by MHCII.

8. Polypeptide according to claims 6 or 7, wherein the immunogen is linked to the GD2 polypeptide via a peptide linker.

9. Polypeptide according to claims 1 to 8, further comprising a cellular uptake enhancing moiety.

10. Polypeptide according to claim 9, wherein the cellular uptake enhancing moiety is selected from the group consisting of human immune deficiency virus (HIV) Tat and cellular uptake enhancing fragments and variants thereof, herpes simplex virus (HSV) VP22 tegument protein and cellular uptake enhancing fragments and variants thereof, homeoproteins including Drosophila homeotic protein antennapedia (ANTp), Engrailed 1 (En1), Engrailed 2 (En2), Hoxa-5, Hoxc-8, Hoxb-4 and KNOTTED-1 and cellular uptake enhancing fragments and variants thereof.

11. Polypeptide comprising a GD2 peptide mimotope, wherein the GD2 peptide mimotope is producible by a method comprising the steps of
(a) synthesizing a polypeptide comprising a GD2 peptide mimotope having an amino acid sequence wherein one or two amino acid(s) from the amino acid sequence selected from the group consisting of SEQ ID NO. 3 to 14 has(have) been changed and
(b) selecting a polypeptide exhibiting a K_{D} to an antibody having an Ig light chain sequence according to SEQ ID 1 and an Ig heavy chain sequence according to SEQ ID NO. 2 of 150 x 10⁻⁶ M or less.

12. Polynucleotide comprising a nucleotide sequence encoding a polypeptide of any of claims 1 to 11.

13. Vector containing the polynucleotide of claim 12.

14. Vector of claim 13, which is an expression vector, a gene targeting vector and/or a gene transfer vector.

15. Vector of claims 13 or 14, wherein the polynucleotide is operatively linked to expression control sequences allowing expression in prokaryotic and/or eukaryotic host cells.

16. Host cell genetically engineered with the polynucleotide of claim 12 or the vector of any of claims 13 to 15.

17. Host cell of claim 16 further comprising a polynucleotide sequence encoding an immunogen.

18. Host cell of claim 17, wherein the immunogen is selected from the group consisting of T1 epitope of HIV gp 120; zymosan; heat shock proteins (HSP), in particular HSP 70; polycationic peptides, in particular poly-L-arginine; fibronectin; flagellin; cytokines with adjuvant activity, in particular GM-CSF, interleukin- (IL-)2, IL-6, IL-7, IL-11, IL-18, type I and II, interferons, in particular interferon-gamma, TNF-alpha; synthetic oligopeptides presented by MHCII.

19. Pharmaceutical composition comprising a polypeptide according to claims 1 to 11, a polynucleotide according to claim 12, a vector according to claim 13 to 15 or a host cell according to claim 16 to 18 and a pharmaceutically acceptable adjuvant.

20. Pharmaceutical composition according to claim 19, further comprising and immunogen.

21. Pharmaceutical composition according to claim 20, wherein the immunogen is selected from the group consisting of unmethylated DNA, in particular unmethylated DNA comprising CpG dinucleotides (CpG motif), in particular CpG ODN with phosphorothioate (PTO) backbone (CpG PTO ODN) or phosphodiester (PO) backbone (CpG PO ODN); bacterial products from the outer membrane of Gram-negative bacteria, in particular monophosphoryl lipid A (MPLA), lipopolysaccharides (LPS), muramyl dipeptides and derivatives thereof; synthetic lipopeptide derivatives, in particular Pam₃Cys; lipoarabinomannan; peptidoglycan; zymosan; heat shock proteins (HSP), in particular HSP 70; dsRNA and synthetic derivatives thereof, in particular Poly I:poly C; polycationic peptides, in particular poly-L-arginine; taxol; fibronectin; flagellin; imidazoquinoline; cytokines with adjuvant activity, in particular GM-CSF, interleukin- (IL-)2, IL-6, IL-7, IL-18, type I and II, interferons, in particular interferon-gamma, TNF-alpha; 25-dihydroxyvitamin D3 (calcitriol); synthetic oligopeptides, in particular MHCII-presented peptides; gel-like precipitates of aluminum hydroxide (alum).

22. Use of a polypeptide according to claims 1 to 11, a polynucleotide according to claim 12, a vector according to claim 13 to 15 or a host cell according to claim 16 to 18 for the production of a medicament for the treatment of tumors of neuroectodermal origin.

23. Use of claim 22, wherein the tumor of neuroectodermal origin is selected from melanoma, neuroblastoma, glioma, soft tissue sarcoma and small cell carcinoma of the lung.


SEQUENCE LISTING

<110> Charité - Universitätsmedizin Berlin

<120> GD2 Peptide mimotopes, their production and use

<130> 522-9

<160> 18

<170> PatentIn version 3.3

<210> 1
<211> 240
<212> PRT
<213> Mus musculus

<400> 1

Met Glu Ala Pro Ala Gln Leu Leu Phe Leu Leu Leu Leu Trp Leu Pro
1               5                   10                  15

Asp Thr Thr Gly Glu Ile Val Met Thr Gln Ser Pro Ala Thr Leu Ser
            20                  25                  30

Val Ser Pro Gly Glu Arg Ala Thr Leu Ser Cys Arg Ser Ser Gln Ser
        35                  40                  45

Leu Val His Arg Asn Gly Asn Thr Tyr Leu His Trp Tyr Leu Gln Lys
    50                  55                  60

Pro Gly Gln Ser Pro Lys Leu Leu Ile His Lys Val Ser Asn Arg Phe
65                  70                  75                  80

Ser Gly Val Pro Asp Arg Phe Ser Gly Ser Gly Ser Gly Thr Asp Phe
                85                  90                  95

Thr Leu Lys Ile Ser Arg Val Glu Ala Glu Asp Leu Gly Val Tyr Phe
            100                 105                 110

Cys Ser Gln Ser Thr His Val Pro Pro Leu Thr Phe Gly Ala Gly Thr
        115                 120                 125

Lys Leu Glu Leu Lys Arg Thr Val Ala Ala Pro Ser Val Phe Ile Phe
    130                 135                 140

Pro Pro Ser Asp Glu Gln Leu Lys Ser Gly Thr Ala Ser Val Val Cys
145                 150                 155                 160

Leu Leu Asn Asn Phe Tyr Pro Arg Glu Ala Lys Val Gln Trp Lys Val
                165                 170                 175

Asp Asn Ala Leu Gln Ser Gly Asn Ser Gln Glu Ser Val Thr Glu Gln
            180                 185                 190

Asp Ser Lys Asp Ser Thr Tyr Ser Leu Ser Ser Thr Leu Thr Leu Ser
        195                 200                 205

Lys Ala Asp Tyr Glu Lys His Lys Val Tyr Ala Cys Glu Val Thr His
    210                 215                 220

Gln Gly Leu Ser Ser Pro Val Thr Lys Ser Phe Asn Arg Gly Glu Cys
225                 230                 235                 240

<210>  2
<211>  462
<212>  PRT
<213>  Mus musculus

<400>  2

Met Gly Trp Thr Trp Ile Phe Ile Leu Ile Leu Ser Val Thr Thr Gly
1               5                   10                  15

Val His Ser Glu Val Gln Leu Leu Gln Ser Gly Pro Glu Leu Glu Lys
            20                  25                  30

Pro Gly Ala Ser Val Met Ile Ser Cys Lys Ala Ser Gly Ser Ser Phe
        35                  40                  45

Thr Gly Tyr Asn Met Asn Trp Val Arg Gln Asn Ile Gly Lys Ser Leu
    50                  55                  60

Glu Trp Ile Gly Ala Ile Asp Pro Tyr Tyr Gly Gly Thr Ser Tyr Asn
65                  70                  75                  80

Gln Lys Phe Lys Gly Arg Ala Thr Leu Thr Val Asp Lys Ser Ser Ser
                85                  90                  95

Thr Ala Tyr Met His Leu Lys Ser Leu Thr Ser Glu Asp Ser Ala Val
            100                 105                 110

Tyr Tyr Cys Val Ser Gly Met Glu Tyr Trp Gly Gln Gly Thr Ser Val
        115                 120                 125

Thr Val Ser Ser Ala Ser Thr Lys Gly Pro Ser Val Phe Pro Leu Ala
    130                 135                 140

Pro Ser Ser Lys Ser Thr Ser Gly Gly Thr Ala Ala Leu Gly Cys Leu
145                 150                 155                 160

Val Lys Asp Tyr Phe Pro Glu Pro Val Thr Val Ser Trp Asn Ser Gly
                165                 170                 175

Ala Leu Thr Ser Gly Val His Thr Phe Pro Ala Val Leu Gln Ser Ser
            180                 185                 190

Gly Leu Tyr Ser Leu Ser Ser Val Val Thr Val Pro Ser Ser Ser Leu
        195                 200                 205

Gly Thr Gln Thr Tyr Ile Cys Asn Val Asn His Lys Pro Ser Asn Thr
    210                 215                 220

Lys Val Asp Lys Arg Val Glu Pro Lys Ser Cys Asp Lys Thr His Thr
225                 230                 235                 240

Cys Pro Pro Cys Pro Ala Pro Glu Leu Leu Gly Gly Pro Ser Val Phe
                245                 250                 255

Leu Phe Pro Pro Lys Pro Lys Asp Thr Leu Met Ile Ser Arg Thr Pro
            260                 265                 270

Glu Val Thr Cys Val Val Val Asp Val Ser His Glu Asp Pro Glu Val
        275                 280                 285

Lys Phe Asn Trp Tyr Val Asp Gly Val Glu Val His Asn Ala Lys Thr
    290                 295                 300

Lys Pro Arg Glu Glu Gln Tyr Asn Ser Thr Tyr Arg Val Val Ser Val
305                 310                 315                 320

Leu Thr Val Leu His Gln Asp Trp Leu Asn Gly Lys Glu Tyr Lys Cys
                325                 330                 335

Lys Val Ser Asn Lys Ala Leu Pro Ala Pro Ile Glu Lys Thr Ile Ser
            340                 345                 350

Lys Ala Lys Gly Gln Pro Arg Glu Pro Gln Val Tyr Thr Leu Pro Pro
        355                 360                 365

Ser Arg Glu Glu Met Thr Lys Asn Gln Val Ser Leu Thr Cys Leu Val
    370                 375                 380

Lys Gly Phe Tyr Pro Ser Asp Ile Ala Val Glu Trp Glu Ser Asn Gly
385                 390                 395                 400

Gln Pro Glu Asn Asn Tyr Lys Thr Thr Pro Pro Val Leu Asp Ser Asp
                405                 410                 415

Gly Ser Phe Phe Leu Tyr Ser Lys Leu Thr Val Asp Lys Ser Arg Trp
            420                 425                 430

Gln Gln Gly Asn Val Phe Ser Cys Ser Val Met His Glu Ala Leu His
        435                 440                 445

Asn His Tyr Thr Gln Lys Ser Leu Ser Leu Ser Pro Gly Lys
    450                 455                 460

<210>  3
<211>  10
<212>  PRT
<213>  Artificial

<220>
<223>  GD2 mimotope

<400>  3

Gly Arg Leu Lys Met Val Pro Asp Leu Glu
1               5                   10

<210>  4
<211>  10
<212>  PRT
<213>  Artificial

<220>
<223>  GD2 mimotope

<400>  4

Gly Arg Leu His Met Val Pro Asp Leu Glu
1               5                   10

<210>  5
<211>  10
<212>  PRT
<213>  Artificial

<220>
<223>  GD2 mimotope

<400>  5

Gly Arg Leu Ser Met Val Pro Asp Leu Glu
1               5                   10

<210>  6
<211>  10
<212>  PRT
<213>  Artificial

<220>
<223>  GD2 mimotope

<400>  6

Gly Arg Leu Lys Leu Val Pro Asp Leu Glu
1               5                   10


<210>  7
<211>  10
<212>  PRT
<213>  Artificial

<220>
<223>  GD2 mimotope

<400>  7

Gly Arg Leu His Leu Val Pro Asp Leu Glu
1               5                   10


<210>  8
<211>  10
<212>  PRT
<213>  Artificial

<220>
<223>  GD2 mimotope

<400>  8

Gly Arg Leu Ser Leu Val Pro Asp Leu Glu
1               5                   10


<210>  9
<211>  10
<212>  PRT
<213>  Artificial

<220>
<223>  GD2 mimotope

<400>  9

Gly Arg Leu Asn Leu Val Pro Asp Leu Glu
1               5                   10


<210>  10
<211>  10
<212>  PRT
<213>  Artificial

<220>
<223>  GD2 mimotope

<400>  10

Asp Gly Gly Trp Leu Ser Lys Gly Ser Trp
1               5                   10


<210>  11

<211>  10
<212>  PRT
<213>  Artificial

<220>
<223>  GD2 mimotope

<400>  11

Asp Gly Gly Trp Leu Ser Glu Gly Ser Trp
1               5                   10


<210>  12
<211>  10
<212>  PRT
<213>  Artificial

<220>
<223>  GD2 mimotope

<400>  12

Asp Gly Trp Trp Leu Ser Glu Gly Ser Trp
1               5                   10


<210>  13
<211>  10
<212>  PRT
<213>  Artificial

<220>
<223>  GD2 mimotope

<400>  13

Asp Gly Asp Trp Leu Ser Lys Gly Ser Trp
1               5                   10


<210>  14
<211>  10
<212>  PRT
<213>  Artificial

<220>
<223>  GD2 mimotope

<400>  14

Asp Gly Gly Trp Leu Ser Asn Gly Ser Trp
1               5                   10


<210>  15
<211>  12
<212>  PRT
<213>  Artificial

<220>
<223>  GD2 mimotope

<400>  15

Cys Gly Arg Leu Lys Met Val Pro Asp Leu Glu Cys
1               5                   10


<210>  16
<211>  12
<212>  PRT
<213>  Artificial

<220>
<223>  GD2 mimotope

<400>  16

Cys Asp Gly Gly Trp Leu Ser Lys Gly Ser Trp Cys
1               5                   10


<210>  17
<211>  12
<212>  PRT
<213>  Artificial

<220>
<223>  GD2 mimotope

<400>  17

Cys Gly Arg Leu His Leu Val Pro Asp Leu Glu Cys
1               5                   10


<210>  18
<211>  11
<212>  PRT
<213>  Artificial

<220>
<223>  random peptide

<400>  18

Ser Ala Thr Pro Trp Asp Leu Lys Thr Ser Leu
1               5                   10